# EUROPÄISCHE PATENTSCHRIFT

(11) **EP 3 748 667 B1**
(45) Veröffentlichungstag und Bekanntmachung des Hinweises auf die Patenterteilung: **08.10.2025**
(21) Anmeldenummer: 19178363.8
(22) Anmeldetag: 05.06.2019
(51) Int. Cl.: H01J 35/06, H05G 1/58

(54) **SILIZIUM-FELDEFFEKT-EMITTER**
SILICON FIELD EFFECT EMITTER
ÉMETTEUR EN SILICIUM À EFFET DE CHAMP

(43) Veröffentlichungstag der Anmeldung: 09.12.2020
(73) Patentinhaber: Siemens Healthineers AG, 91301 Forchheim (DE)
(72) Erfinder: Freudenberger, Jörg, 90562 Kalchreuth (DE); Fritzler, Anja, 91052 Erlangen (DE)
(74) Vertreter: Siemens Healthineers Patent Attorneys

(56) Entgegenhaltungen:
- US-A1- 2009 185 660
- US-A1- 2010 239 064
- US-A1- 2010 260 317
- "ADVANCES IN ELECTRONICS AND ELECTRON PHYSICS.", vol. 83, 1 January 1992, ACADEMIC PRESS INC., NEW YORK, NY., US, ISSN: 0065-2539, article I. BRODIE ET AL: "Vacuum Microelectronics", pages: 1 - 106, XP055647180, DOI: 10.1016/S0065-2539(08)60006-2

## Beschreibung

Die Erfindung betrifft eine Röntgenröhre, eine Röntgeneinrichtung, ein Verfahren für ein Generieren von Röntgenstrahlung in einer vorgegebenen Ortsverteilung auf einer Anode und ein zugehöriges Computerprogrammprodukt.

Üblicherweise wird in einer Röntgenröhre Röntgenstrahlung durch einen Beschuss einer Anode mit Elektronen erzeugt. Ein Auflösungsvermögen der Röntgenröhre wird üblicherweise von einer Ortsverteilung der Elektronen beim Auftreffen auf der Anode bestimmt. Die Elektronen werden herkömmlicherweise von einem thermionischen Wolframemitter, einem Kohlenstoff-Feldeffekt-Emitter und/oder oder einem Feldeffekt-Emitter mit einer Dispensorkathode emittiert. Den vorher beschriebenen Emittern ist gemein, dass eine Elektronemissionsdichte üblicherweise bei ungefähr 3 A/cm^2 begrenzt ist. Für eine bildgebende Untersuchung ist allerdings üblicherweise eine Elektronenemissionsdichte von ungefähr 10 A/cm^2 erforderlich, weshalb die Elektronen der herkömmlichen Emitter üblicherweise auf die Anode insbesondere mittels einer Ablenkeinheit fokussiert werden. Das Fokussieren der Elektronen hängt typischerweise von einer Beschleunigungsspannung zwischen einer Kathode mit einem Emitter und einer Anode sowie einer Raumladungsdichte der Elektronen ab. Üblicherweise ändert sich also die Ortsverteilung der Elektronen in Abhängigkeit der Beschleunigungsspannung und/oder einer Intensität des Elektronenstrahls.

Aus der EP 3 531 437 A1 ist eine Elektronen-Emissionsvorrichtung mit wenigstens einen Elektronen-Emitter und mit wenigstens einem Sperrgitter bekannt.

Die EP 3 518 266 A1 offenbart eine thermionische Emissionsvorrichtung mit einem Flachemitter und mit einem zuschaltbaren Feldeffekt-Elektronenemitter.

Guerrera et al. beschreiben einen Silizium-Feldeffekt-Emitter mit einer Elektronenemissionsdichte über 100 A/cm^2 in "Silicon Field Emitter Arrays With Current Densities Exceeding 100 A/cm2 at Gate Voltages Below 75 V" (IEEE ELECTRON DEVICE LETTERS, VOL. 37, NO. 1, JANUARY 2016).

US 2009 / 0 185 660 A1 und US 2010 / 0 260 317 A1 offenbaren eine multifokale Röntgenstrahlenquelle basierend auf einer Vielzahl an Feldeffektemittereinheiten. Aus der US 2010 / 0 239 064 A1 ist eine Computertomographie mit einer Vielzahl an Röntgenstrahlenquellen, welche in unterschiedlichen Winkeln angeordnet sind, bekannt. Brodie et al. beschäftigen sich mit dem Einsatz von mikroelektronischen Einheiten in Vakuumgeräten (ADVANCES IN ELECTRONICS AND ELECTRON PHYSICS, VOL. 83, ISBN 0-12-014725-4).

Der Erfindung liegt die Aufgabe zu Grunde, eine Röntgenröhre, eine Röntgeneinrichtung, ein Verfahren für ein Generieren von Röntgenstrahlung in einer vorgegebenen Ortsverteilung auf einer Anode und ein zugehöriges Computerprogrammprodukt anzugeben, bei welchen die Emission der Elektronen flexibler gesteuert wird.

Die Aufgabe wird durch die Merkmale der unabhängigen Ansprüche gelöst. Vorteilhafte Ausgestaltungen sind in den Unteransprüchen beschrieben.

Das nach Anspruch 1 erfindungsgemäße Verfahren für ein Generieren von Röntgenstrahlung in einer vorgegebenen Ortsverteilung auf einer Anode einer Röntgenröhre weist folgende Schritte auf:
- Vorgeben der Ortsverteilung von auf der Anode einer Röntgenröhre auftreffenden Elektronen,
- Auswählen einer Gruppe an mehreren Feldeffekt-Emitternadeln eines Emitters der Röntgenröhre in Abhängigkeit von einer Beschleunigungsspannung in der Steuereinheit,
- Anschalten der ausgewählten Gruppe an mehreren Feldeffekt-Emitternadeln, wodurch Röntgenstrahlung in der vorgegebenen Ortsverteilung auf der Anode generiert wird.

Die Ortsverteilung entspricht insbesondere einer Modulations-Transfer-Funktion. Das Vorgeben der Ortsverteilung kann beispielsweise mittels Eingabemittel, insbesondere einer Tastatur, einer Maus und/oder eines Bildschirms, durch den Nutzer und/oder Arzt erfolgen. Alternativ kann beispielsweise eine Steuereinheit die Ortsverteilung automatisch ermitteln, insbesondere in Abhängigkeit des von dem Nutzer und/oder Arzt vorgegebenen Messprotokolls.

Das Auswählen der Gruppe erfolgt vorzugsweise automatisch, in der Steuereinheit, insbesondere in Abhängigkeit des von dem Nutzer und/oder Arzt vorgegebenen Messprotokolls.

Das Anschalten der ausgewählten Gruppe an mehreren Feldeffekt-Emitternadeln kann ein Anlegen einer Gate-Emitter-Spannung umfassen. Durch das Anschalten der ausgewählten Gruppe an mehreren Feldeffekt-Emitternadeln werden Elektronen emittiert und derart auf die Anode ausgerichtet, dass beim Auftreffen der Elektronen in der vorgegebenen Ortsverteilung auf der Anode die Röntgenstrahlung generiert wird.

Die Röntgenröhre weist
- eine Anode,
- eine erste Schaltvorrichtung,
- eine zweite Schaltvorrichtung,
- eine Steuereinheit und
- einen Emitter mit mehreren Feldeffekt-Emitternadeln auf,
wobei zumindest eine Feldeffekt-Emitternadel der mehreren Feldeffekt-Emitternadeln einen Durchmesser von weniger als 1 µm und Silizium aufweist,
wobei eine erste Gruppe der mehreren Feldeffekt-Emitternadeln mittels der ersten Schaltvorrichtung anschaltbar oder ausschaltbar ist,
wobei eine zweite Gruppe der mehreren Feldeffekt-Emitternadeln mittels der zweiten Schaltvorrichtung anschaltbar oder ausschaltbar ist,
wobei sich die erste Gruppe von der zweiten Gruppe in einer Anordnung und in der Anzahl der mehreren Feldeffekt-Emitternadeln sowie in der angelegten Beschleunigungsspannung unterscheidet, wobei die erste Gruppe und die zweite Gruppe zumindest teilweise dieselben Feldeffekt-Emitternadeln aufweisen und
wobei die Steuereinheit zum Ansteuern der ersten Schaltvorrichtung und der zweiten Schaltvorrichtung ausgebildet ist. Der Emitter mit den mehreren Feldeffekt-Emitternadeln ist vorzugsweise gemäß dem Silizium-Feldeffekt-Emitter von Guerrera et al. ausgebildet. Die zumindest eine Feldeffekt-Emitternadel kann insbesondere einen Durchmesser zwischen 10 nm und 800 nm, vorzugsweise zwischen 100 nm und 500 nm, besonders vorteilhafterweise zwischen 150 nm und 250 nm, beispielsweise 200 nm aufweisen. Typischerweise weist die zumindest eine Feldeffekt-Emitternadel keinen Kohlenstoff und lediglich Silizium auf. Grundsätzlich ist es denkbar, dass jede feldeffekt-Emitternadel Silizium aufweist und/oder aus Silizium besteht. Der Emitter weist pro Quadratmillimeter insbesondere zwischen 2 und 25000000, vorzugsweise zwischen 100000 und 10000000 Feldeffekt-Emitternadeln auf. Die mehreren Feldeffekt-Emitternadeln weisen typischerweise einen Abstand zueinander zwischen 10 nm und 500 µm, vorzugsweise zwischen 200 nm und 1 µm auf. Die mehreren Feldeffekt-Emitternadeln sind üblicherweise parallel ausgerichtet.

Üblicherweise kann die zumindest eine Feldeffekt-Emitternadel, vorzugsweise jede Feldeffekt-Emitternadel, mit einer Gate-Emitter-Spannung insbesondere größer 0V, kleiner 1 kV, vorzugsweise kleiner 200 V, besonders vorteilhafterweise kleiner 100 V angeschaltet werden. Insbesondere die Gate-Emitter-Spannung erzwingt eine derartige elektrische Feldstärke an einer Spitze der angeschalteten Feldeffekt-Emitternadeln, so dass Elektronen von den angeschalteten Feldeffekt-Emitternadeln emittiert werden. Das Ausschalten der ersten Gruppe und/oder der zweiten Gruppe kann insbesondere mit einem Anlegen einer Gate-Emitter-Spannung gleich oder kleiner 0V erfolgen.

Die Anode kann insbesondere eine Stehanode oder eine Drehanode sein. Die Anode weist beispielsweise Wolfram zur Generierung von Röntgenstrahlung aus den Elektronen auf. Die Drehanode kann Teil einer Drehkolbenröntgenröhre oder einer Drehanodenröntgenröhre sein. Die Röntgenröhre ist typischerweise evakuiert. Zur Beschleunigung der von den angeschalteten Feldeffekt-Emitternadeln emittierten Elektronen ist typischerweise eine Beschleunigungsspannung zwischen dem Emitter und der Anode angelegt. Die Beschleunigungsspannung wird beispielsweise von einer Spannungsversorgung bereitgestellt. Die Röntgenröhre weist beispielsweise die Spannungsversorgung zum Anlegen einer ersten Beschleunigungsspannung oder einer zweiten Beschleunigungsspannung zwischen dem Emitter und der Anode auf. Die erste Beschleunigungsspannung kann sich von der zweiten Beschleunigungsspannung unterscheiden oder gleich der zweiten Beschleunigungsspannung sein. Die Steuereinheit steuert in Abhängigkeit der angelegten Beschleunigungsspannung die erste Schaltvorrichtung oder die zweite Schaltvorrichtung an.

Die mehreren Feldeffekt-Emitternadeln werden gruppenweise geschaltet. Die erste Gruppe und die zweite Gruppe unterscheiden sich, typischerweise in zumindest einer Feldeffekt-Emitternadel. Die erste Gruppe und die zweite Gruppe weisen zumindest teilweise dieselben Feldeffekt-Emitternadeln auf.

Die erste Gruppe und die zweite Gruppe sind nicht gleich.

Die mehreren Feldeffekt-Emitternadeln sind typischerweise in zwei Raumrichtungen verteilt und/oder rasterförmig angeordnet. Die Anordnung der mehreren Feldeffekt-Emitternadeln ist üblicherweise derart, dass der Emitter ein zweidimensionaler Emitter ist. Vorteilhafterweise sind die mehreren Feldeffekt-Emitternadeln in einer Ebene angeordnet und/oder parallel ausgerichtet. Grundsätzlich ist es denkbar, dass die mehreren Feldeffekt-Emitternadeln linear oder mehrdimensional angeordnet sind.

Es ist denkbar, dass jede Feldeffekt-Emitternadel jeweils einen Schalter aufweist, welcher mit der ersten Schaltvorrichtung und/oder der zweiten Schaltvorrichtung verbunden ist. Der Schalter kann eine Feldeffekt-Emitternadel-Spannungsversorgung für das Bereitstellen der Gate-Emitter-Spannung sein. Die erste Schaltvorrichtung und die zweite Schaltvorrichtung können insbesondere logisch in Programmcodemitteln abgebildet sein, während insbesondere die Schalter der mehreren Feldeffekt-Emitternadeln elektronisch die Emission von Elektronen an der jeweiligen Feldeffekt-Emitternadeln schalten können. Insbesondere wenn die erste Schaltvorrichtung und die zweite Schaltvorrichtung logisch abgebildet sind, kann vorzugsweise die erste Gruppe und die zweite Gruppe wiederholt mit den mehreren Feldeffekt-Emitternadeln belegt werden. In anderen Worten kann vorteilhafterweise die Steuereinheit die Feldeffekt-Emitternadeln für die erste Gruppe und/oder die Feldeffekt-Emitternadeln für die zweite Gruppe beispielsweise in Abhängigkeit eines Messprotokolls auswählen. In diesem Fall ist vorzugsweise die Röntgenröhre für die verschiedenen Messprotokolle einsetzbar, ohne dass die Feldeffekt-Emitternadeln erneut elektronisch verschaltet werden. Die Gruppen der mehreren Feldeffekt-Emitternadeln sind insbesondere mittels der Steuereinheit derart ansteuerbar, dass die Steuereinheit die erste Schaltvorrichtung und/oder die zweite Schaltvorrichtung mit den jeweiligen Schaltern der ersten Gruppe und/oder der zweiten Gruppe programmiert.

Das Schalten der Emission der Elektronen kann ein vollständiges Sperren, insbesondere beim Abschalten, und/oder ein stufenloses Regulieren, insbesondere beim Anschalten, der Emission der Elektronen umfassen. Das stufenlose Regulieren der Emission der Elektronen kann einem Einstellen eines Emissionsstroms der jeweiligen angeschalteten Feldeffekt-Emissionsnadel entsprechen. Das stufenlose Regulieren kann von der Gate-Emitter-Spannung abhängen.

Das Anschalten und das Ausschalten, vorzugsweise das zeitliche versetzte gruppenbezogene Schalten, der Feldeffekt-Emitternadeln kann insbesondere mit einer Frequenz von höher 0.1 Hz, vorteilhafterweise höher 10 kHz, besonders vorteilhafterweise höher 1 MHz wiederholt erfolgen. Das im Vergleich zu einem thermionischen Emitter hochfrequente Anschalten und Ausschalten ist insbesondere vorteilhaft, weil die Wärmeverteilung innerhalb des Emitters effizient gesteuert werden kann und/oder eine Gesamtanzahl der emittierten Elektronen, insbesondere ein Gesamtemissionsstrom als Summe der Emissionsströme, flexibel eingestellt werden kann.

Die erste Schaltvorrichtung ist mit der ersten Gruppe, beispielsweise über die Schalter der jeweiligen Feldeffekt-Emitternadeln der ersten Gruppe, verknüpft und die zweite Schaltvorrichtung ist mit der zweiten Gruppe, beispielsweise über die Schalter der jeweiligen Feldeffekt-Emitternadeln der zweiten Gruppe, verknüpft. Die Verknüpfung entspricht insbesondere einer elektronischen Verschaltung. Die Steuereinheit ist insbesondere zu einem derartigen Ansteuern der ersten Schaltvorrichtung und der zweiten Schaltvorrichtung ausgebildet, dass mittels eines Aussendens eines ersten Anschaltsignals beispielsweise die erste Schaltvorrichtung die erste Gruppe der mehreren Feldeffekt-Emitternadeln, insbesondere deren Schalter, anschaltet und/oder mittels eines Aussendens eines zweiten Anschaltsignals beispielsweise die zweite Schaltvorrichtung die zweite Gruppe der mehreren Feldeffekt-Emitternadeln, insbesondere deren Schalter, anschaltet. Die erste Schaltvorrichtung kann die Feldeffekt-Emitternadeln der ersten Gruppe gemäß dem Anschaltsignal anschalten, beispielsweise indem die Schalter der Feldeffekt-Emitternadeln der ersten Gruppe angeschaltet werden. Das Ansteuern umfasst als Alternative zum Anschalten zusätzlich das Ausschalten, beispielsweise durch das Aussenden eines Ausschaltsignals.

Vorteilhafterweise kann die derartig ausgebildete Röntgenröhre die für eine bildgebende Untersuchung erforderliche Elektronenemissionsdichte, insbesondere von ungefähr 10 A/cm^2, vorzugsweise auf der Anode bereitstellen. Dabei ist nur ein Teil, beispielsweise die erste Gruppe oder die zweite Gruppe, der mehreren Feldeffekt-Emitternadeln angeschaltet. Wenn nicht alle Feldeffekt-Emitternadeln angeschaltet sind, ist dies beispielsweise für eine Lebensdauer der Röntgenröhre, insbesondere für den Emitter mit den mehreren Feldeffekt-Emitternadeln, vorteilhaft, insbesondere wenn eine Wärmeentwicklung innerhalb des Emitters homogen verteilt ist. Die längere Lebensdauer ist insbesondere in Hinblick auf eine Kostenreduktion vorteilhaft.

Vorteilhafterweise weist die Röntgenröhre keine Elektronenstrahl-Ablenkeinheit zwischen dem Emitter und der Anode auf. Eine herkömmliche Elektronenstrahl-Ablenkeinheit weist üblicherweise eine magnetische Ablenkeinheit auf. Die erste Gruppe und/oder die zweite Gruppe werden vorteilhafterweise derart in Anzahl und/oder Anordnung ausgewählt, dass die Röntgenröhre keine Elektronenstrahl-Ablenkeinheit zwischen dem Emitter und der Anode benötigt. Dadurch ist insbesondere ein Gewicht der Röntgenröhre niedriger als ein Gewicht einer herkömmlichen Röntgenröhre mit Ablenkeinheit. Üblicherweise ist die Röntgenröhre in diesem Fall günstiger als die herkömmliche Röntgenröhre. Weiterhin ist vorzugsweise eine Steuerung des Elektronenstrahls vereinfacht, wenn die Röntgenröhre weniger komplex aufgrund der fehlenden Ablenkeinheit ist.

Dass die mehreren Feldeffekt-Emitternadeln in Gruppen unterteilt und/oder geschaltet werden können, ermöglicht insbesondere das Anschalten der Feldeffekt-Emitternadeln in Abhängigkeit von verschiedenen Messprotokollen für die bildgebende Untersuchung, wobei vorzugsweise die Ortsverteilung der Elektronen auf der Anode unverändert ist. Die verschiedenen Messprotokolle können sich insbesondere auch in einer Röntgenstrahlendosis und/oder in einem Auflösungsvermögen unterscheiden. Die mehreren Feldeffekt-Emitternadeln der ersten Gruppe und/oder die mehreren Feldeffekt-Emitternadeln der zweiten Gruppe können beispielsweise von einem Hersteller der Röntgenröhre und/oder einem Nutzer und/oder einem Arzt der Röntgenröhre festgelegt werden. Das Festlegen der Feldeffekt-Emitternadeln für die jeweilige Gruppe kann einem Programmieren des Emitters entsprechen. Vorteilhafterweise können zusätzlich zu der ersten Gruppe und zu der zweiten Gruppe weitere Gruppen an Feldeffekt-Emitternadeln festgelegt werden. Vorzugsweise kann die Steuereinheit in Abhängigkeit des Messprotokolls eine Anzahl an Schaltungsvorrichtungen und die der jeweiligen Schaltungsvorrichtung gruppenweise zugeordneten Feldeffekt-Emitternadeln festlegen.

Weiterhin ermöglicht das gruppenweise Anschalten der Feldeffekt-Emitternadeln ein flexibles Formen des Elektronenstrahls. Besonders vorteilhafterweise sind die Feldeffekt-Emitternadeln der ersten Gruppe und die Feldeffekt-Emitternadeln der zweiten Gruppe derart angeordnet, dass eine vorgegebene Ortsverteilung der auf der Anode auftreffenden Elektronen im Wesentlichen unabhängig von der angeschalteten Gruppe ist.

Die erste Gruppe unterscheidet sich von der zweiten Gruppe in einer Anordnung der mehreren Feldeffekt-Emitternadeln. Die Anordnung ist insbesondere in Bezug auf einen Referenzpunkt nicht deckungsgleich. Wenn sich die Feldeffekt-Emitternadeln der beiden Gruppen in der Anordnung unterscheiden, wird üblicherweise die Form des Elektronenstrahls insbesondere durch physikalische Wechselwirkungen der emittierten Elektronen zueinander beeinflusst und damit die Ortsverteilung der Elektronen. Das Berücksichtigen der physikalischen Wechselwirkungen ermöglicht vorzugsweise das Weglassen der Ablenkeinheit. Die physikalischen Wechselwirkungen umfassen insbesondere elektrostatische Effekte aufgrund der Raumladungsdichte der Elektronen. Die Anordnung der angeschalteten Feldeffekt-Emitternadeln kann beispielsweise stern- oder kreisförmig oder beliebig sein.

Die erste Gruppe unterscheidet sich von der zweiten Gruppe in der Anzahl der mehreren Feldeffekt-Emitternadeln. Wenn die Anzahl variiert, ist üblicherweise automatisch ein Unterschied in der Anordnung vorhanden. Die Variation der Anzahl der mehreren Feldeffekt-Emitternadeln der jeweiligen Gruppe ermöglicht vorzugsweise das Skalieren des Emissionsstroms.

Die erste Gruppe unterscheidet sich von der zweiten Gruppe in der angelegten Beschleunigungsspannung. Dies ist insbesondere vorteilhaft, weil in Abhängigkeit der Beschleunigungsspannung die Wärmeverteilung über die Feldeffekt-Emitternadeln verteilt werden kann.

Die Anordnung und/oder die Anzahl der Feldeffekt-Emitternadeln und/oder die angelegte Beschleunigungsspannung kann beispielsweise in einer Simulation der Röntgenröhre oder bei einer Testmessung mit der Röntgenröhre ermittelt werden und/oder gruppenspezifisch in einer Speichereinheit der Steuereinheit abgespeichert werden.

Erfindungsgemäß ist also vorgesehen, dass sich die erste Gruppe von der zweiten Gruppe in der Anzahl sowie der Anordnung der mehreren Feldeffekt-Emitternadeln sowie der angelegten Beschleunigungsspannung unterscheidet. Dadurch können vorteilhafterweise die physikalischen Wechselwirkungen zwischen den emittierten Elektronen, insbesondere aufgrund der Raumladungsdichte, derart berücksichtigt werden, dass die Ortsverteilung der Elektronen auf der Anode unabhängig von der angeschalteten Schaltungsvorrichtung gleich ist. In anderen Worten treffen die Elektronen in diesem Ausführungsbeispiel vorteilhafterweise in derselben Ortsverteilung auf der Anode auf, unabhängig davon, welche Feldeffekt-Emitternadeln angeschaltet sind. Ein weiterer Vorteil kann sein, dass die Röntgenstrahlendosis der mittels der Elektronen generierten Röntgenstrahlung unabhängig von den angeschalteten Feldeffekt-Emitternadeln ist, wobei die Röntgenstrahlendosis typischerweise von der angelegten Beschleunigungsspannung abhängt. Die Röntgenstrahlendosis entspricht insbesondere einer Intensität der Röntgenstrahlung.

Es ist denkbar, dass die erste Schaltvorrichtung und/oder die zweite Schaltvorrichtung zu einem derartigen Anschalten der jeweiligen mehreren Feldeffekt-Emitternadeln ausgebildet sind, dass jede angeschaltete Feldeffekt-Emitternadel einen Sättigungsstrom liefert. Dies ist insbesondere vorteilhaft, da sich die erste Gruppe von der zweiten Gruppe in der Anzahl der mehreren Feldeffekt-Emitternadeln unterscheidet. In diesem Fall korreliert typischerweise der Gesamtemissionsstrom des Emitters mit der Anzahl der angeschalteten Feldeffekt-Emitternadeln. Der Gesamtemissionsstrom kann insbesondere flexibel gesteuert werden. Gemäß Guerrera et al. kann der Sättigungstrom durch einen spezifischen Aufbau der mehreren Feldeffekt-Emitternadeln bestimmt werden, wobei der spezifische Aufbau insbesondere einen effektive Querschnittsfläche und/oder eine Dotierungsdichte der jeweiligen Feldeffekt-Emitternadel und/oder eine Sättigungsgeschwindigkeit der Elektronen umfasst. Das Betreiben der Feldeffekt-Emitternadeln in Sättigung ist insbesondere vorteilhaft, weil dadurch der Emissionsstrom der jeweiligen angeschalteten Feldeffekt-Emitternadel konstant gehalten wird. In anderen Worten umfasst das Ansteuern der ersten Schaltvorrichtung und der zweiten Schaltvorrichtung ein binäres Schalten der ersten Gruppe oder der zweiten Gruppe, insbesondere ohne stufenlose Stromregelung. In Sättigung liefert die jeweilige angeschaltete Feldeffekt-Emitternadel einen konstanten Strom, unabhängig von dem angelegten elektrischen Feld. Die jeweilige angeschaltete Feldeffekt-Emitternadel weist insbesondere eine Strombegrenzung auf. Im Vergleich zu einem Silizium-Feldeffekt-Emitter kann ein herkömmlicher Kohlenstoff-Feldeffekt-Emitter durch ein zu großes elektrisches Feld zerstört werden, weil der herkömmliche Kohlenstoff-Feldeffekt-Emitter typischerweise keine Strombegrenzung aufweist. In dieser Ausführungsform ist der Emissionsstrom typischerweise nicht stufenlos einstellbar. Die Gate-Emitter-Spannung ist vorzugsweise größer oder gleich einer Sättigungsspannung.

Die erfindungsgemäße Röntgeneinrichtung weist die Röntgenröhre und den Röntgendetektor auf. Mittels des Röntgendetektors kann vorteilhafterweise die bei einer bildgebenden Untersuchung einen Patienten durchleuchtende Röntgenstrahlung erfasst werden. Beispielsweise kann mittels der erfassten Röntgenstrahlung ein medizinisches Bild rekonstruiert werden. Das medizinische Bild kann beispielsweise dem Nutzer oder dem Arzt auf einer Anzeigeeinheit bereitgestellt und/oder in einem Radiologieinformationssystem und/oder in einem PACS-Bildarchivierungssystem abgespeichert werden. Die Röntgeneinrichtung kann insbesondere als ein konventionelles Röntgensystem, als ein Single-Quellen-Computertomograph, als ein Mammographiesystem oder als ein C-Bogen-Angiographiesystem ausgebildet sein.

Eine Ausführungsform sieht vor, dass die Röntgeneinrichtung für eine bildgebende Untersuchung mit wechselnder Beschleunigungsspannung ausgebildet ist. Die wechselnde Beschleunigungsspannung ist insbesondere gemäß dem Dual-Energy-Messprotokoll vorgegeben. Die Beschleunigungsspannung wechselt vorzugsweise in einer Frequenz größer 1 Hz, vorzugsweise größer 100 Hz, besonders vorteilhafterweise größer 1 kHz. Ein weiterer Vorteil dieser Ausführungsform ist, dass die wechselsende Beschleunigungsspannung mittels der jeweiligen Gruppe der Feldeffekt-Emitternadeln schnell geschaltet werden kann. In diesem Ausführungsbeispiel können beispielsweise Materialien innerhalb des Patienten unterschieden werden, wobei die Materialien üblicherweise einen Schwächungskoeffizienten als Funktion der Beschleunigungsspannung aufweisen. Die Materialien können insbesondere Gewebe und/oder Knochen und/oder Kontrastmittel sein. Die Röntgeneinrichtung weist insbesondere lediglich eine einzige Röntgenröhre auf, vorzugsweise keine weitere Röntgenröhre wie bei einer herkömmlichen Dual-Energie-Röntgeneinrichtung. In anderen Worten wird für die bildgebende Untersuchung gemäß dem Dual-Energy-Messprotokoll keine weitere Röntgenröhre benötigt.

Das Computerprogrammprodukt kann ein Computerprogramm sein oder ein Computerprogramm umfassen. Das Computerprogrammprodukt weist die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist derart konfiguriert, dass die Recheneinheit mittels des Computerprogrammprodukts die erfindungsgemäßen Verfahrensschritte ausführt. Die Programmcodemittel können in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 einen Emitter mit einer ersten Gruppe der mehreren Feldeffekt-Emitternadeln ,
Fig. 2 einen Emitter mit einer zweiten Gruppe der mehreren Feldeffekt-Emitternadeln,
Fig. 3 Trajektorien der emittierten Elektronen in Abhängigkeit der angelegten Beschleunigungsspannung,
Fig. 4 eine Röntgeneinrichtung in einem nicht erfindungsgemäßen Fall und
Fig. 5 ein Verfahren für ein Generieren von Röntgenstrahlung in einer vorgegebenen Ortsverteilung auf einer Anode.

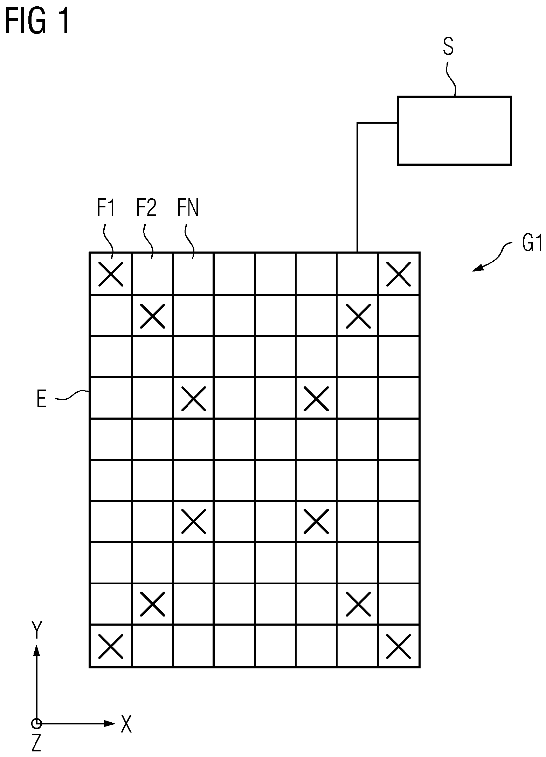

**Fig. 1** zeigt eine Draufsicht auf einen Emitter E mit einer ersten Gruppe G1 der mehreren Feldeffekt-Emitternadeln F1, F2, FN. Die mehreren Feldeffekt-Emitternadeln F1, F2, FN sind in zwei Raumrichtungen verteilt angeordnet und umfassen in diesem Ausführungsbeispiel 8x10 Feldeffekt-Emitternadeln.

Die erste Gruppe G1 der mehreren Feldeffekt-Emitternadeln F1, F2, FN ist mit Kreuzchen gekennzeichnet und umfasst in diesem Ausführungsbeispiel 12 Feldeffekt-Emitternadeln. Die erste Anzahl ist also 12. Grundsätzlich ist es denkbar, dass die erste Gruppe mehr oder weniger als 12 Feldeffekt-Emitternadeln aufweist. Wenn die Steuereinheit S eine nicht in Fig. 1 gezeigte Schaltvorrichtung ansteuert und anschaltet, emittiert die erste Gruppe G1 der mehreren Feldeffekt-Emitternadeln F1, F2, FN Elektronen. Diese Elektronen werden von einer ersten Beschleunigungsspannung beschleunigt, welche beispielsweise zwischen 30 kV und 150 kV, vorzugsweise bei 120 kV liegt.

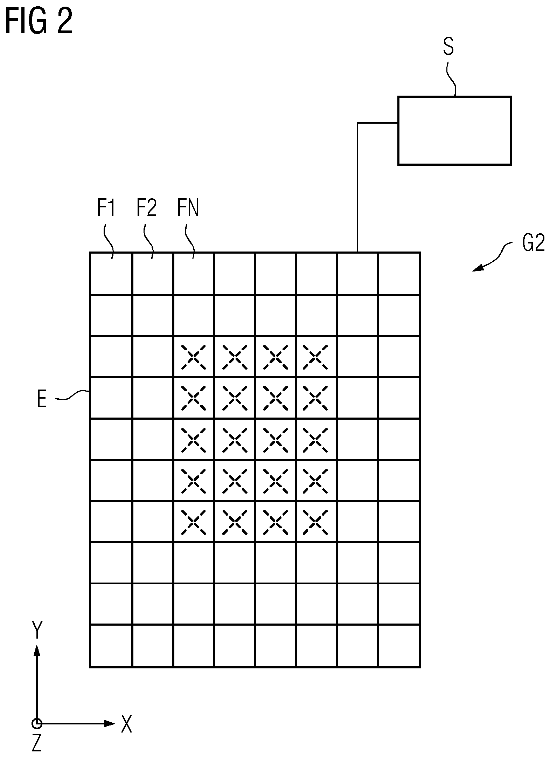

Die erste Gruppe G1 weist eine erste Anordnung und die erste Anzahl der mehreren Feldeffekt-Emitternadeln F1, F2, FN auf, welche sich von dem in Fig. 2 Gezeigten unterscheidet.

**Fig. 2** zeigt eine Draufsicht auf einen Emitter E mit einer zweiten Gruppe G2 der mehreren Feldeffekt-Emitternadeln F1, F2, FN. Die zweite Gruppe G2 unterscheidet sich von der ersten Gruppe G1. Die zweite Gruppe G2 weist eine zweite Anordnung und eine zweite Anzahl der mehreren Feldeffekt-Emitternadeln F1, F2, FN auf. Die zweite Anzahl ist 20. Die zweite Gruppe G1 der mehreren Feldeffekt-Emitternadeln F1, F2, FN ist mit gestrichelten Kreuzchen gekennzeichnet.

Die aus der zweiten Gruppe G2 emittierten Elektronen werden von einer zweiten Beschleunigungsspannung (die sich erfindungsgemäß von der ersten Beschleunigungsspannung unterscheidet) beschleunigt, welche beispielsweise zwischen 30 kV und 150 kV, vorzugsweise bei 80 kV liegt.

Die in Fig. 2 angeschalteten Feldeffekt-Emitternadeln der zweiten Gruppe G2 sind kompakter angeordnet als die in Fig. 1 angeschalteten Feldeffekt-Emitternadeln der ersten Gruppe G1. Die in Fig. 1 und Fig. 2 gezeigten Gruppen G1, G2 der mehreren Feldeffekt-Emitternadeln können zeitlich versetzt, insbesondere nacheinander, angeschaltet werden, beispielsweise gemäß dem Dual-Energy-Messprotokoll.

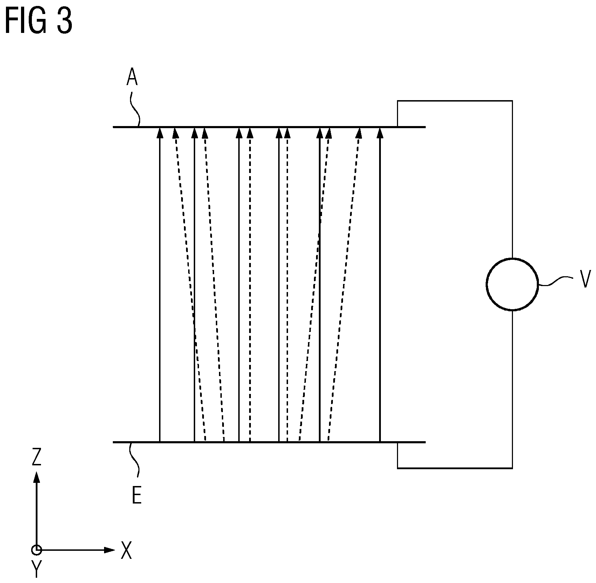

**Fig. 3** zeigt schematisch Trajektorien der emittierten Elektronen in Abhängigkeit der zeitlich versetzt angelegten Beschleunigungsspannung. Die Elektronen werden insbesondere emittiert, wenn die Gate-Emitter-Spannung mittels einer nicht in Fig. 3 gezeigten Feldeffekt-Emitternadel-Spannungsversorgung entsprechend bereitgestellt ist. Die vom Emitter E emittierten Elektronen werden mittels einer Spannungsversorgung V zu einer Anode A hin vorzugsweise in einem Vakuum beschleunigt.

Die durchgezogenen Trajektorien unterscheiden sich von den gestrichelten Trajektorien in der Anzahl der emittierenden Feldeffekt-Emitternadeln und damit in dem Effekt der physikalischen Wechselwirkungen zueinander. Die durchgezogenen Trajektorien zeigen beispielsweise Elektronen der in Fig. 1 gezeigten Ausgestaltung und die gestrichelten Trajektorien der in Fig. 2 gezeigten Ausgestaltung, wenn die erste Anzahl der Fig. 1 kleiner ist als die zweite Anzahl der Fig. 2. Aufgrund des höheren Emissionsstroms und der einhergehenden höheren Raumladungsdichte der in Fig. 2 gezeigten Ausführungsform, wodurch insbesondere vergleichsweise stärkere physikalische Wechselwirkungen zwischen den Elektronen auftreten, laufen die gestrichelten Trajektorien stärker auseinander als die durchgezogenen Trajektorien.

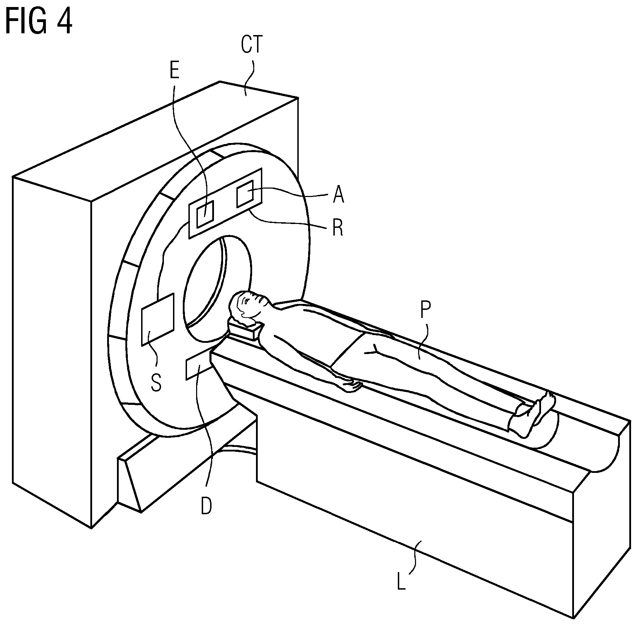

**Fig. 4** zeigt eine Röntgenröhre R in einer nicht der Erfindung entsprechenden Anordnung. Die Röntgenröhre R weist eine Anode A, eine erste Schaltvorrichtung, eine zweite Schaltvorrichtung, eine Steuereinheit S und einen Emitter E mit mehreren Feldeffekt-Emitternadeln F1, F2, FN auf. Zumindest eine Feldeffekt-Emitternadel der mehreren Feldeffekt-Emitternadeln F1, F2, FN weist einen Durchmesser von weniger als 1 µm und Silizium auf. Eine erste Gruppe G1 der mehreren Feldeffekt-Emitternadeln F1, F2, FN ist mittels der ersten Schaltvorrichtung anschaltbar oder ausschaltbar. Eine zweite Gruppe G2 der mehreren Feldeffekt-Emitternadeln F1, F2, FN ist mittels der zweiten Schaltvorrichtung anschaltbar oder ausschaltbar. Die erste Gruppe G1 unterscheidet sich von der zweiten Gruppe G2. Die Steuereinheit S ist zum Ansteuern der ersten Schaltvorrichtung und der zweiten Schaltvorrichtung ausgebildet. Die Steuereinheit S ist erfindungsgemäß Teil der Röntgenröhre R. Die Steuereinheit S kann insbesondere einen FPGA oder einen Prozessor aufweisen. In diesem hier dargestellten, nicht erfindungsgemäßen Fall ist die Steuereinheit S außerhalb der Röntgenröhre R angeordnet und mit der Röntgenröhre R zum Ansteuern der ersten Schaltvorrichtung und der zweiten Schaltvorrichtung verbunden.

Grundsätzlich ist es denkbar, dass die erste Schaltvorrichtung und/oder die zweite Schaltvorrichtung zu einem derartigen Anschalten der jeweiligen mehreren Feldeffekt-Emitternadeln F1, F2, FN ausgebildet sind, dass jede angeschaltete Feldeffekt-Emitternadel einen Sättigungsstrom liefert.

Die Röntgeneinrichtung weist die Röntgenröhre R und einen Röntgendetektor D auf, wobei die Röntgeneinrichtung für eine bildgebende Untersuchung mit wechselnder Beschleunigungsspannung ausgebildet ist. Die Beschleunigungsspannung wechselt während der bildgebenden Untersuchung, vorzugsweise gemäß dem Dual-Energy-Messprotokoll. Die Röntgeneinrichtung ist als Teil eines Single-Quellen-Computertomograph CT gezeigt. Ein Patient P ist auf einer Patientenliege L gelagert.

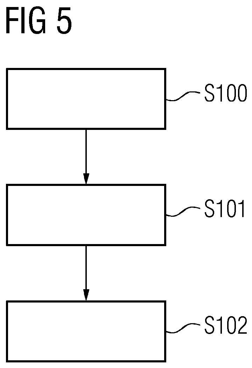

**Fig. 5** zeigt ein Flussdiagramm eines Verfahrens für ein Generieren von Röntgenstrahlung in einer vorgegebenen Ortsverteilung auf einer Anode.

Verfahrensschritt S100 kennzeichnet ein Vorgeben der Ortsverteilung von auf der Anode einer Röntgenröhre auftreffenden Elektronen.

Verfahrensschritt S101 kennzeichnet ein Auswählen einer Gruppe an mehreren Feldeffekt-Emitternadeln eines Emitters der Röntgenröhre in Abhängigkeit von einer Beschleunigungsspannung.

Verfahrensschritt S102 kennzeichnet ein Anschalten der ausgewählten Gruppe an mehreren Feldeffekt-Emitternadeln, wodurch Röntgenstrahlung in der vorgegebenen Ortsverteilung auf der Anode generiert wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen, der ausschließlich durch die Ansprüche definiert ist.

## Patentansprüche

1. Verfahren für ein Generieren von Röntgenstrahlung in einer vorgegebenen Ortsverteilung auf einer Anode (A) einer Röntgenröhre (R), wobei die Röntgenröhre (R) ferner eine erste Schaltvorrichtung, eine zweite Schaltvorrichtung, eine Steuereinheit (S) und einen Emitter (E) mit mehreren Feldeffekt-Emitternadeln (F1, F2, FN) aufweist, wobei zumindest eine Feldeffekt-Emitternadel der mehreren Feldeffekt-Emitternadeln (F1, F2, FN) einen Durchmesser von weniger als 1 µm und Silizium aufweist, wobei eine erste Gruppe (G1) der mehreren Feldeffekt-Emitternadeln (F1, F2, FN) mittels der ersten Schaltvorrichtung anschaltbar oder ausschaltbar ist, wobei eine zweite Gruppe (G2) der mehreren Feldeffekt-Emitternadeln (F1, F2, FN) mittels der zweiten Schaltvorrichtung anschaltbar oder ausschaltbar ist, wobei die erste Gruppe (Gl) und die zweite Gruppe (G2) zumindest teilweise dieselben Feldeffekt-Emitternadeln aufweisen,
wobei sich die erste Gruppe (G1) von der zweiten Gruppe (G2) in einer Anordnung und in der Anzahl der mehreren Feldeffekt-Emitternadeln (F1, F2, FN) sowie in der angelegten Beschleunigungsspannung unterscheidet,
wobei die Steuereinheit (S) zum Ansteuern der ersten Schaltvorrichtung und der zweiten Schaltvorrichtung ausgebildet ist,
umfassend die Schritte:
- Vorgeben der Ortsverteilung von auf der Anode (A) auftreffenden Elektronen,
- Auswählen einer Gruppe (G1, G2) in Abhängigkeit von einer Beschleunigungsspannung in der Steuereinheit (S),
- Anschalten der ausgewählten Gruppe (G1, G2) an mehreren Feldeffekt-Emitternadeln (F1, F2, FN), wodurch Röntgenstrahlung in der vorgegebenen Ortsverteilung auf der Anode (A) generiert wird.

2. Röntgenröhre (R), eingerichtet zur Durchführung des Verfahrens nach Anspruch 1.

3. Röntgeneinrichtung, aufweisend
- die Röntgenröhre (R) nach Anspruch 2 und
- einen Röntgendetektor (D).

4. Röntgeneinrichtung nach Anspruch 3, wobei die Röntgeneinrichtung für eine bildgebende Untersuchung mit wechselnder Beschleunigungsspannung ausgebildet ist.

5. Computerprogrammprodukt, welches direkt in einen Speicher einer Recheneinheit der Steuereinheit der Röntgenröhre nach Anspruch 2 ladbar ist, mit Programmcodemitteln, um ein Verfahren nach Anspruch 1 auszuführen, wenn das Computerprogrammprodukt in der Recheneinheit ausgeführt wird.

## Claims

1. Method for generating X-ray radiation in a predefined spatial distribution on an anode (A) of an X-ray tube (R), wherein the X-ray tube (R) furthermore has a first switching device, a second switching device, a control unit (S) and an emitter (E) with multiple field effect emitter needles (F1, F2, FN), wherein at least one field effect emitter needle of the multiple field effect emitter needles (F1, F2, FN) has a diameter of less than 1 µm and silicon, wherein a first group (G1) of the multiple field effect emitter needles (F1, F2, FN) can be activated or deactivated by means of the first switching device, wherein a second group (G2) of the multiple field effect emitter needles (F1, F2, FN) can be activated or deactivated by means of the second switching device, wherein the first group (G1) and the second group (G2) have at least partially the same field effect emitter needles, wherein the first group (G1) differs from the second group (G2) in an arrangement and in the number of the multiple field effect emitter needles (F1, F2, FN) as well as in the acceleration voltage applied, wherein the control unit (S) is designed to actuate the first switching device and the second switching device,
comprising the steps:
- predefining the spatial distribution of electrons striking the anode (A),
- selecting a group (G1, G2) as a function of an acceleration voltage in the control unit (S),
- activating the selected group (G1, G2) of multiple field effect emitter needles (F1, F2, FN), as a result of which X-ray radiation is generated in the predefined spatial distribution on the anode (A).

2. X-ray tube (R), configured to perform the method according to claim 1.

3. X-ray device, having
- the X-ray tube (R) according to claim 2 and
- an X-ray detector (D).

4. X-ray device as claimed in claim 3, wherein the X-ray device is designed for an imaging examination with an alternating acceleration voltage.

5. Computer program product which can be loaded directly into a memory of a computer unit of the control unit of the X-ray tube according to claim 2, having program code means in order to carry out a method according to claim 1 when the computer program product is executed in the computer unit.

## Revendications

1. Procédé de production de rayonnement X en une répartition locale donnée à l'avance sur une anode (A) d'un tube (R) à rayons X, dans lequel le tube (R) à rayons X a, outre un premier dispositif de coupure, un deuxième dispositif de coupure, une unité (S) de commande et un émetteur (E) ayant plusieurs aiguilles (F1, F2, FN) d'émetteur à effet de champ, dans lequel au moins une aiguille d'émetteur à effet de champ des plusieurs aiguilles (F1, F2, FN) d'émetteur à effet de champ a un diamètre de moins de 1 µm et du silicium, dans lequel un premier groupe (G1) des plusieurs aiguilles (F1, F2, FN) d'émetteur à effet de champ peut être mis en circuit ou mis hors circuit au moyen du premier dispositif de coupure, dans lequel un deuxième groupe (G2) des plusieurs aiguilles (F1, F2, FN) d'émetteur à effet de champ peut être mis en circuit ou hors circuit au moyen du deuxième dispositif de coupure, dans lequel le premier groupe (G1) et le deuxième groupe (G2) ont au moins en partie les mêmes aiguilles d'émetteur à effet de champ, dans lequel le premier groupe (G1) se distingue du deuxième groupe (G2) par un agencement et par le nombre des plusieurs aiguilles (F1, F2, FN) d'émetteur à effet de champ, ainsi que par la tension d'accélération appliquée,
dans lequel l'unité (S) de commande est constituée pour commander le premier dispositif et le deuxième dispositif de coupure, comprenant les stades :
- prescription de la répartition locale d'électrons arrivant sur l'anode (A),
- sélection d'un groupe (G1, G2) en fonction d'une tension d'accélération dans l'unité (S) de commande,
- mise en circuit du groupe (G1, G2) sélectionné de plusieurs aiguilles (F1, F2, FN) d'émetteur à effet de champ, grâce à quoi du rayonnement X est produit sur l'anode (A) en la répartition locale donnée à l'avance.

2. Tube (R) à rayons X, agencé pour effectuer le procédé suivant la revendication 1.

3. Dispositif à rayons X, comportant
- le tube (R) à rayons X de la revendication 2 et
- un détecteur (D) de rayons X.

4. Dispositif à rayons X suivant la revendication 3, dans lequel le dispositif à rayons X est constitué pour un examen donnant une image avec une tension d'accélération alternée.

5. Produit de programme d'ordinateur, qui peut être chargé directement dans la mémoire d'une unité informatique de l'unité de commande du tube à rayons X suivant la revendication 2, comprenant des moyens de code de programme, pour exécuter un procédé suivant la revendication 1, lorsque le produit de programme d'ordinateur est exécuté dans l'unité informatique.
